# EUROPÄISCHE PATENTSCHRIFT

(11) **EP 0 604 726 B1**
(45) Veröffentlichungstag und Bekanntmachung des Hinweises auf die Patenterteilung: **24.01.1996**
(21) Anmeldenummer: 93117234.0
(22) Anmeldetag: 25.10.1993
(51) Int. Cl.: C07D 233/18, C07C 219/08, C11D 1/62, D06M 13/46

(54) **Verfahren zur Quaternierung von Triethanolaminfettsäureestern und Imidazolinamiden in alkoxylierten Fetten oder Ölen als Reaktionsmedium und die Verwendung der Reaktionsmischungen als Wäscheweichspülerwirkstoffkomponenten**
Process for the quaternisation of fatty acid esters of triethanolamine and imidazolinamides in alkoxylated fats or oils as the reaction medium and the use of the reaction mixture as a fabric conditioner component
Procédé de quaternisation d'esters triéthanolaminiques d'acides gras et d'imidazolinamides avec des graisses et huiles alcoxylées comme milieu réactionnel et l'utilisation des melanges réactionnels comme composants d'un agent adoucissant

(30) Priorität: 23.12.1992 DE 4243862
(43) Veröffentlichungstag der Anmeldung: 06.07.1994
(73) Patentinhaber: HÜLS AKTIENGESELLSCHAFT, D-45764 Marl (DE)
(72) Erfinder: Brock, Michael, Dr., D-46514 Schermbeck (DE); Hardt, Peter, Dr., D-40789 Monheim (DE); Klimmek, Helmut, Dr., D-47803 Krefeld (DE); Stockhausen, Dolf, Dr., D-47800 Krefeld (DE)

(56) Entgegenhaltungen:
- EP-A- 0 284 036
- EP-A- 0 295 385
- EP-A- 0 431 652
- EP-A- 0 569 847
- WO-A-91/17974

## Beschreibung

Die Erfindung betrifft ein Verfahren zur Quaternierung von Triethanolaminfettsäureestern und Imidazolinamiden in alkoxylierten natürlichen Fetten oder Ölen oder deren Mischungen mit freien Fettsäuren, Mono- und/oder Diglyceriden (im folgenden allgemein als alkoxylierte Fette bezeichnet) als Reaktionsmedium und die Verwendung der erhaltenen Reaktionsmischungen als Wäscheweichspülerwirkstoffkomponenten.

Es ist bekannt, quaternierte Fettsäureester des Triethanolamins (N-Alkyl-N,N,N-trihydroxyethylammoniumfettsäureester-Salze, im folgenden mit Esterquat bezeichnet) bzw. quaternierte Imidazolinamide (1-Alkyl-2-alkyl-3-alkylamidoimidazolinium-Salze, im folgenden mit Imidazoliniumquat bezeichnet) als Wirkstoffkomponente in Wäscheweichspülern einzusetzen. Nähere Beschreibungen sowohl der Herstellung als auch der Verwendung der Ester- und Imidazoliniumquats (im folgenden allgemein als Quats bezeichnet) als Wäscheweichspülerwirkstoffkomponenten finden sich in EP 0 295 385, DE 37 10 064, WO 91/17974, US 3 915 867, BE 888 678, DD 159 263 und EP 0 431 652.

Als Quaternierungsmittel finden Benzylhalogenide, Methylhalogenide, Dimethylsulfat oder Dipropylsulfat Verwendung. Die Quaternierung von Triethanolaminfettsäureestern bzw. Imidazolinamiden erfolgt in niedermolekularen Alkoholen, vorzugsweise in 2-Propanol, als Lösungsmittel. 2-Propanol wird nach der Quaternierung nicht entfernt, so daß die Quats als 85 - 90%ige Ware vermarktet werden (s. o. g. Schutzrechte). Der Zusatz von 2-Propanol sichert eine bei der Temperatur der Quaternierung (40 - 95 °C) fließfähige Konsistenz sowohl der Reaktionsmischungen als auch der entstehenden Quats. Dies ist aus produktionstechnischen Gründen von Wichtigkeit. Des weiteren ist dadurch die Weiterverarbeitung der Quats z. B. zu Wäscheweichspülerfertigformulierungen gewährleistet. Das eingesetzte 2-Propanol fungiert also ausschließlich als Verdünner und hat keinen Zusätzlichen weichmachenden Einfluß im Zusammenwirken mit den Quats. Der Einsatz von 2-Propanol ist außerdem problematisch, weil sein Flammpunkt (12 °C) sehr niedrig ist.

Die DE-OS 42 15 689 beschreibt die Verwendung von alkoxylierten Fetten als alleinige Wäscheweichspülerwirkstoffkomponenten oder in Abmischungen mit den üblichen stickstoffhaltigen kationenaktiven Wirkstoffkomponenten wie N,N-Distearyl-N,N-dimethylammoniumsalzen, quaternierten bzw. protonierten Imidazolinderivaten, quaternierten Fettsäureestern des Triethanolamins sowie 2,3-Dihydroxypropyl-1-trimethylammoniumsalz-Derivaten. Deren Quaternierung erfolgt wie üblich in 2-Propanol als Lösungsmittel. Bei Verwendung von Abmischungen von alkoxylierten Fetten mit stickstoffhaltigen kationenaktiven Wirkstoffkomponenten, wie z. B. Esterquats und Imidazoliniumquats, werden diese in 2-Propanol, in der Regel als 90%ige Konzentrate, eingesetzt.

Es war Aufgabe der Erfindung, die Quaternierung von Triethanolaminfettsäureestern bzw. Imidazolinamiden in einem Reaktionsmedium durchzuführen, das neben der fließfähigen Beschaffenheit der Reaktionsmischung einen synergistischen Effekt im Hinblick auf die weichmachende Wirkung von Quats ausübt. Das Gemisch aus Medium und Quat sollte also eine im Vergleich zu den Quats in 2-Propanol bessere Weichmachung bei gleicher Einsatzmenge bewirken. Zusätzlich soll der Flammpunkt des Mediums möglichst größer als 100 °C sein.

Die Aufgabe wird erfindungsgemäß durch die Verwendung von alkoxylierten Fetten als Medium anstelle von 2-Propanol gelöst. Zu den Fetten gehören grundsätzlich alle Triglyceride und deren Mischungen mit freien Fettsäuren, Mono- und/oder Diglyceriden. Die Alkoxylierung kann gemäß DE-OS 36 17 657 und DE-OS 38 26 179 erfolgen.

Beispielhaft werden für die Alkoxylierung Ethylenoxid, Propylenoxid und Butylenoxid genannt. Verwendet man mehr als ein Epoxid, so können diese entweder nacheinander oder gleichzeitig mit den Fetten umgesetzt werden.

Die alkoxylierten Fette werden vor der Quaternierung der Triethanolaminfettsäureester bzw. Imidazolinamide zu diesen in einem Verhältnis von 1 : 99 bis 90 : 10, bevorzugt 5 : 95 bis 80 : 20, zugesetzt. Die Quaternierung erfolgt unter den üblichen Bedingungen mit Benzylhalogeniden, Methylhalogeniden, Dimethylsulfat oder Dipropylsulfat. Bevorzugt verwendet wird Dimethylsulfat.

Gegenstand der Erfindung ist daher ein Verfahren zur Quaternierung von Triethanolaminfettsäureestern und Imidazolinamiden mit Benzylhalogeniden, Methylhalogeniden, Dimethylsulfat oder Dipropylsulfat, dadurch gekennzeichnet, daß als Reaktionsmedium alkoxylierte natürliche Fette oder Öle oder deren Mischungen mit freien Fettsäuren, Mono- und/oder Diglyceriden eingesetzt werden.

Weiterer Gegenstand der Erfindung ist die Verwendung der erhaltenen Reaktionsmischungen aus den quaternierten Triethanolaminfettsäureestern bzw. Imidazolinamiden und den alkoxylierten natürlichen Fetten oder Ölen oder deren Mischungen mit freien Fettsäuren, Mono- und/oder Diglyceriden als Wäscheweichspülerwirkstoffkomponenten. Der Anteil der Reaktionsprodukte an der Wäscheweichspülerzusammensetzung beträgt 1 bis 50 Gew.-%, bevorzugt 5 - 30 Gew.-%, bei Zusatz der üblichen Additive wie Elektrolyt, Emulgatoren, Dispergatoren, Duft- und Farbstoffe sowie Wasser.

Wie überraschenderweise gefunden wurde, kann nach Ersatz der bisher üblichen Quats in 2-Propanol durch die beanspruchten Quats in alkoxylierten Fetten bei gleicher weichmachender Wirkung die Menge der Quats um bis zu 68 % reduziert werden. Die eingesetzten alkoxylierten Fette weisen durchweg Flammpunkte von größer als 100 °C auf.

Die nachfolgenden Beispiele sollen die Ansprüche beispielhaft belegen, ohne diese einzuschränken. Die angegebenen Ansätze stellen hellfarbene pastöse Substanzen dar, die einen Schmelzbereich von 20 - 50 °C aufweisen.

### Herstellung von Quats in alkoxylierten Fetten

### Beispiel 1: Herstellung von Esterquats in propoxyliertem Palmöl

In einem 250 ml Dreihalskolben, ausgestattet mit Intensivkühler, Tropftrichter und Kontaktthermometer werden Triethanolaminfettsäureester (Veresterungsgrad: 1,5, C-Kettenverteilung der zugrundeliegenden Fettsäure: 3 Gew.-%, C₁₄, 38 Gew.-% C₁₆, 3 Gew.-% C₁₆-en, 9 Gew.-% C₁₈, 47 Gew.-% C₁₈-en) und das Reaktionsprodukt aus der Umsetzung von Palmöl mit 15 Gew.-% Propylenoxid ("propoxyliertes Palmöl") vorgelegt. Nach dem Spülen der Apparatur mit Stickstoff wird auf 60 °C erwärmt und unter ständigem Rühren innerhalb von 15 min. Dimethylsulfat zugetropft. Die Temperatur steigt vorübergehend auf 90 - 95 °C an. Nach 10stündigem Rühren bei 60 °C wird kein Dimethylsulfat mehr nachgewiesen (Nachweis mit Hilfe von Drägerröhrchen der Fa. Dräger, untere Nachweisgrenze 0,005 ppm).

| | Ansatz | | |
|---|---|---|---|
| | A | B | C |
| Einsatzmenge Triethanolaminfettsäureester [g] | 100 | 100 | 45 |
| Einsatzmenge propoxyliertes Palmöl [g] | 11,1 | 17,7 | 105,0 |
| Gewichtsverhältnis von Triethanolaminfettsäureester zu propoxyliertem Palmöl | 90 : 10 | 85 : 15 | 30 : 70 |
| Einsatzmenge Dimethylsulfat [g] | 21,3 | 21,3 | 9,6 |

### Beispiel 2: Herstellung von Esterquats in propoxyliertem Hautfett

Die Versuchsdurchführung erfolgt analog der im Beispiel 1 beschriebenen mit dem Unterschied, daß in diesem Beispiel das Reaktionsprodukt aus der Umsetzung von Hautfett mit 15 Gew.-% Propylenoxid ("propoxyliertes Hautfett") statt des propoxylierten Palmöls eingesetzt wird.

| | Ansatz | | |
|---|---|---|---|
| | D | E | F |
| Einsatzmenge Triethanolaminfettsäureester [g] | 100 | 100 | 45 |
| Einsatzmenge propoxyliertes Hautfett [g] | 11,1 | 17,7 | 105,0 |
| Gewichtsverhältnis von Triethanolaminfettsäureester zu propoxyliertem Hautfett | 90 : 10 | 85 : 15 | 30 : 70 |
| Einsatzmenge Dimethylsulfat [g] | 21,3 | 21,3 | 9,6 |

### Beispiel 3: Herstellung von Imidazoliniumquats in propoxyliertem Palmöl

Der Versuchsablauf ist analog dem in Beispiel 1 beschriebenen. Es werden 2-Alkyl-3-alkylamidoethylimidazolin (C-Kettenverteilung der zugrundeliegenden Fettsäure: 3 Gew.-% C₁₄, 38 Gew.-% C₁₆, 3 Gew.-% C₁₆-en, 9 Gew.-% C₁₈, 47 Gew.-% C₁₈-en) und das Reaktionsprodukt aus der Umsetzung von Palmöl und 15 Gew.-% Propylenoxid ("propoxyliertes Palmöl") vorgelegt. Nach dem Spülen der Apparatur mit Stickstoff wird auf 60 °C erwärmt und unter ständigem Rühren innerhalb von 15 min. Dimethylsulfat zugetropft. Die Temperatur steigt vorübergehend auf 90 - 95 °C an. Nach 10stündigem Rühren bei 60 °C wird kein Dimethylsulfat mehr nachgewiesen (Nachweis siehe Beispiel 1).

| | Ansatz | | |
|---|---|---|---|
| | G | H | K |
| Einsatzmenge 2-Alkyl-3-alkylamidoethylimidazolin [g] | 135,0 | 127,5 | 45,0 |
| Einsatzmenge propoxyliertes Palmöl [g] | 15,0 | 22,5 | 105,0 |
| Gewichtsverhältnis von 2-Alkyl-3-alkylamidoethylimidazolin zu propoxyliertem Palmöl | 90 : 10 | 85 : 15 | 30 : 70 |
| Einsatzmenge Dimethylsulfat [g] | 24,9 | 23,4 | 8,3 |

### Beispiel 4: Herstellung von Imidazoliniumquats in propoxyliertem Hautfett

Die Versuchsdurchführung erfolgt analog der in Beispiel 3 beschriebenen mit dem Unterschied, daß in diesem Beispiel das Reaktionsprodukt aus der Umsetzung von Hautfett mit 15 Gew.-% Propylenoxid ("propoxyliertes Hautfett") statt des propoxylierten Palmöls eingesetzt wird.

| | Ansatz | | |
|---|---|---|---|
| | L | M | N |
| Einsatzmenge 2-Alkyl-3-alkylamidoethylimidazolin [g] | 135,0 | 127,5 | 45,0 |
| Einsatzmenge propoxyliertes Hautfett [g] | 15,0 | 22,5 | 105,0 |
| Gewichtsverhältnis von 2-Alkyl-3-alkylamidoethylimidazolin zu propoxyliertem Hautfett | 90 : 10 | 85 : 15 | 30 : 70 |
| Einsatzmenge Dimethylsulfat [g] | 24,9 | 23,4 | 8,3 |

### Beschreibung der Testmethode der weichmachenden Wirkung

Die weichmachende Wirkung der Ansätze A bis N aus den Beispielen 1 bis 4 wird wie folgt bestimmt: Eine Charge von 72 Stück Frotteehandtüchern (44 cm x 30 cm, ca. 60 g, von der WFK-Testgewebe GmbH) wird, auch wenn nicht alle 72 Tücher gebraucht werden, (aus Gründen gleichmäßiger Abnutzung) einmal mit 100 g eines handelsüblichen Vollwaschmittels (PERSIL, Fa. Henkel) maschinell bei 95 °C gewaschen, gespült und geschleudert. Anschließend folgt ein Waschgang bei 95 °C ohne Waschmittel einschließlich kurzem Anschleudern, so daß saubere, feuchte, etwa das 2,5fache ihres Trockengewichtes an Wasser enthaltene Tücher für manuelles Weichspülen entnommen werden können.

Für einen Versuchsplan analog Tabelle A werden jeweils 9 Tücher in 9 Spülflotten mit dem Standard (= S, MARLOSOFT E 90 ¹⁾ für die Testung der Ansätze A bis F bzw. MARLOSOFT IQ 90 ²⁾ für die Testung der Ansätze G bis N) und 9 Tücher in 9 Spülflotten mit der Testsubstanz (= T, Ansätze A bis N) weichgespült. Dazu werden in Plastikschüsseln jeweils 2 Liter Leitungswasser (12° dH) als Spülflotte und 0,7 g S (Aktivsubstanz berechnet), also 0,35 g S pro Liter Spülflotte, bzw. eine frei zu wählende Menge der Testsubstanz vordispergiert und die feuchten Tücher 10 min. darin belassen. Nach 5 min. werden die Tücher einmal gewendet. Die weichgespülten Tücher werden einzeln jeweils 30 sek. geschleudert und auf dem Wäscheständer in ruhender Luft getrocknet.
1) MARLOSOFT E 90: Esterquat, 90%ig in 2-Propanol, Fa. Hüls
2) MARLOSOFT IQ 90: Imidazoliniumquat, 90%ig in 2-Propanol, Fa. Hüls

**Tabelle A**

| Versuchsplan für die sensorische Weichgriffprüfung mit 6 Prüfern | |
|---|---|
| Prüfer | Tücherkombination mit verschlüsselter Kennzeichnung |
| 1 | SST |
| 2 | SST |
| 3 | SST |
| 4 | STT |
| 5 | STT |
| 6 | STT |
| S = Vergleichssubstanz (Standard), T = Erfindung (Testsubstanz) | |

Den 6 Prüfern werden jeweils 3 Tücher in verschlüsselter Form nach dem Versuchsplan gemäß Tabelle A vorgelegt. Die Aufgabe für den Prüfer besteht darin, das abweichend behandelte Tuch nach sensorischer Begutachtung herauszufinden. Ist dies möglich, vermerkt der Prüfer, ob sich das abweichend behandelte Tuch weicher oder härter anfühlt. Das Gesamtergebnis wird mit Hilfe einer Punkteskala von -6 bis +6 ausgedrückt. Findet z. B. Prüfer 1 das mit T abweichend behandelte Tuch heraus und empfindet es als weicher als die beiden anderen Tücher, so gibt es 1 Pluspunkt. Empfindet er es als härter, so gibt es 1 Minuspunkt. Ist für den Prüfer zwischen den 3 Tüchern kein Unterschied feststellbar, so gibt es 0 Punkte. Ist der Prüfer 1 der Ansicht, daß ein mit S behandeltes Tuch das von den anderen abweichende ist, so gibt es 0,5 Pluspunkte, wenn er dies als härter beurteilt, und 0,5 Minuspunkte, wenn er es als weicher beurteilt. Analog wurde bei den anderen Prüfern verfahren. Anschließend werden die Punkte zusammengezählt. Ist die Summe der Punkte gleich 0, so besteht im Weichgriff zwischen den mit S und T behandelten Tüchern kein Unterschied. Ist die Summe der Punkte größer 0, so ist der Griff der mit T behandelten Tücher weicher als der der mit S behandelten Tücher. Ist die Summe der Punkte kleiner 0, so verhält es sich umgekehrt.

### Beispiel 5: Testung der Weichmachung der in den Beispielen 1 und 2 beschriebenen Ansätze A bis F (Standard: MARLOSOFT E 90)

In Versuchsreihen werden Frotteehandtücher in Spülflotten weichgespült, die aus unterschiedlichen Konzentrationen der in den Beispielen 1 und 2 beschriebenen Ansätze A bis F bestehen. Die Spülflottenkonzentration wird solange variiert, bis die behandelten Tücher sensorisch als ebenso weich beurteilt werden, wie die mit MARLOSOFT E 90 behandelten Tücher. Als Ergebnis der sensorischen Weichgriffprüfung muß somit die Summe aller Punkte gleich 0 sein. Die folgende Aufstellung zeigt die Konzentrationen der Ansätze A bis F, die notwendig sind, um den gleichen Weichgriff zu erhalten, den 0,35 g MARLOSOFT E 90 (berechnet auf den Aktivgehalt) pro Liter Spülflotte bewirken.

| Ansatz | Gesamtkonzentration Ansatz [g/l] | Esterquatanteil [g/l] | Ersparnis Esterquat im Vergleich zum Standard [%] |
|---|---|---|---|
| A | 0,33 | 0,30 | 14 |
| B | 0,30 | 0,27 | 23 |
| C | 0,46 | 0,14 | 60 |
| D | 0,31 | 0,28 | 20 |
| E | 0,28 | 0,24 | 31 |
| F | 0,60 | 0,18 | 48 |

Es zeigt sich in eindrucksvoller Weise, daß der Anteil des benötigten Esterquats bei allen Ansätzen z. T. drastisch gesenkt werden kann. Die eingesparte Menge des Esterquats beträgt bis zu 60 %. Für die Ansätze A, B, D und E gilt zusätzlich, daß sogar die eingesetzte Gesamtkonzentration der Ansätze, also unter Berücksichtigung der Menge an propoxyliertem Palmöl bzw. Hautfett geringer ist, als der eingesetzte Esterquatanteil von 0,35 g des Standards pro Liter Spülflotte.

### Beispiel 6: Testung der Weichmachung der in den Beispielen 3 und 4 beschriebenen Ansätze G bis N (Standard: MARLOSOFT IQ 90)

Es wird analog der in Beispiel 5 beschriebenen Prozedur vorgegangen. Anstelle des Standards MARLOSOFT E 90 wird der Standard MARLOSOFT IQ 90 verwendet.

| Ansatz | Gesamtkonzentration Ansatz [g/l] | Imidazoliniumquatanteil [g/l] | Ersparnis Imidazoliniumquat im Vergleich zum Standard [%] |
|---|---|---|---|
| G | 0,27 | 0,24 | 31 |
| H | 0,29 | 0,25 | 28 |
| K | 0,37 | 0,11 | 68 |
| L | 0,30 | 0,27 | 22 |
| M | 0,28 | 0,24 | 31 |
| N | 0,40 | 0,12 | 65 |

Ähnlich wie im Beispiel 5, zeigt sich auch hier, daß der Anteil des benötigten Imidazoliniumquats bei Zusatz von propoxyliertem Palmöl bzw. Hautfett bei gleichem weichmachendem Effekt um bis zu 68 % gesenkt werden kann.

Für die Ansätze G, H, L und M gilt zusätzlich, daß sogar die eingesetzte Gesamtkonzentration der Ansätze, also unter Berücksichtigung der Menge an propoxyliertem Palmöl bzw. Hautfett geringer ist, als der eingesetzte Imidazoliniumquatanteil von 0,35 g des Standards pro Liter Spülflotte.

## Patentansprüche

1. Verfahren zur Quaternierung von Triethanolaminfettsäureestern und Imidazolinamiden mit Benzylhalogeniden, Methylhalogeniden, Dimethylsulfat oder Dipropylsulfat,
dadurch gekennzeichnet,
daß als Reaktionsmedium alkoxylierte natürliche Fette oder Öle oder deren Mischungen mit freien Fettsäuren, Mono- und/oder Diglyceriden eingesetzt werden.

2. Verfahren nach Anspruch 1,
dadurch gekennzeichnet,
daß das Verhältnis von alkoxylierten Fetten, Ölen oder deren Mischungen mit freien Fettsäuren, Mono- und/oder Diglyceriden zu Triethanolaminfettsäureestern oder Imidazolinamiden 1 : 99 bis 90 : 10 beträgt.

3. Verfahren nach Anspruch 1,
dadurch gekennzeichnet,
daß das Verhältnis von alkoxylierten Fetten, Ölen oder deren Mischungen mit freien Fettsäuren, Mono- und/oder Diglyceriden zu Triethanolaminfettsäureestern oder Imidazolinamiden 5 : 95 bis 80 : 2 beträgt.

4. Verwendung der nach Anspruch 1 erhaltenen Reaktionsmischungen aus den quaternierten Triethanolaminfettsäureestern bzw. Imidazolinamiden und den alkoxylierten natürlichen Fetten oder Ölen oder deren Mischungen mit freien Fettsäuren, Mono- und/oder Diglyceriden als Wäscheweichspülerwirkstoffkomponenten.

5. Verwendung nach Anspruch 4,
dadurch gekennzeichnet,
daß der Anteil der Reaktionsmischungen an der Wäscheweichspülerzusammensetzung 1 bis 50 % beträgt.

6. Verwendung nach Anspruch 4,
dadurch gekennzeichnet,
daß der Anteil der Reaktionsmischungen an der Wäscheweichspülerzusammensetzung 5 bis 30 % beträgt.

## Claims

1. A process for the quaternisation of triethanolamine fatty acid esters and imidazolinamides with benzyl halides, methyl halides, dimethyl sulphate or dipropyl sulphate, characterised in that alkoxylated naturally occuring fats or oils or mixtures thereof with free fatty acids, monoglycerides and/or diglycerides are employed as the reaction medium.

2. A process according to claim 1, characterised in that the ratio of alkoxylated fats, oils or mixtures thereof with free fatty acids, monoglycerides and/or diglycerides to triethanolamine fatty acid esters or imidazolinamides is from 1 : 99 to 90 : 10.

3. A process according to claim 1, characterised in that the ratio of alkoxylated fats, oils or mixtures thereof with free fatty acids, monoglycerides and/or diglycerides to triethanolamine fatty acid esters or imidazolinimides is from 5 : 95 to 80 : 20.

4. Use of the reaction mixtures obtained according to claim 1 from the quaternised triethanolamine fatty acid eaters or imidazolinamides and the alkoxylated naturally occurring fats or oils or mixtures thereof with free fatty acids, monoglyceride: and/or diglycerides as laundry softener active compound components.

5. Use according to claim 4, characterised in that the content of the reaction mixtures in the laundry softener composition is from 1 to 50%.

6. Use according to claim 4, characterised in that the content of the reaction mixtures in the laundry softener composition is from 5 to 30%.

## Revendications

1. Procédé de quaternisation d'esters d'acides gras de triéthanolamine et d'imidazolinamides avec des halogénures de benzyle, des halogénures de méthyle, du sulfate de diméthyle ou du sulfate de dipropyle, caractérisé en ce qu'on utilise comme milieu réactionnel des graisses ou huiles naturelles alcoxylées ou leurs mélanges avec des mono- et/ou des triglycérides ou des acides gras libres.

2. Procédé selon la revendication 1, caractérisé en ce que le rapport des graisses ou huiles alcoxylées ou de leurs mélanges avec des mono- et/ou des triglycérides ou acides gras libres aux esters d'acides gras de triéthanolamine ou aux imidazolinamides est de 1 : 99 à 90 : 10.

3. Procédé selon la revendication 1, caractérisé en ce que le rapport des graisses ou huiles alcoxylées ou de leurs mélanges avec des mono- et/ou des triglycérides ou acides gras libres aux esters d'acides gras de tri-éthanolamine ou aux imidazolinamides est de 5 : 95 à 80 : 2.

4. Utilisation des mélanges réactionnels obtenus selon la revendication 1, constitués des esters d'acides gras de triéthanolamine ou d'imizolinamides quaternisés et des graisses ou huiles naturelles alcoxylées ou de leurs mélanges avec des mono- et/ou des triglycérides ou acides gras, comme matières actives pour produits de rinçage adoucissant pour le linge.

5. Utilisation selon la revendication 4, caractérisée en ce que la quantité des mélanges réactionnels dans la composition de rinçage adoucissant pour le linge est de 1 à 50 %.

6. Utilisation selon la revendication 4, caractérisé en ce que la quantité des mélanges réactionnels dans la composition de rinçage adoucissant pour le linge est de 5 à 30 %.
